# EUROPEAN PATENT SPECIFICATION

(11) **EP 0 804 396 B1**
(45) Date of publication and mention of the grant of the patent: **17.02.1999**
(21) Application number: 95938431.4
(22) Date of filing: 06.11.1995
(51) Int. Cl.: C07C 29/149, C07C 31/125

(54) **PROCESS FOR THE DIRECT HYDROGENATION OF CARBOXYLIC ESTERS**
VERFAHREN ZUM DIREKTEN HYDRIERUNG VON CARBONSÄUREESTERN
PROCEDE D'HYDROGENATION DIRECTE D'ESTERS CARBOXYLIQUES

(30) Priority: 07.11.1994 US 335018; 07.11.1994 US 335021; 07.11.1994 US 335024
(43) Date of publication of application: 05.11.1997
(73) Proprietor: SHELL INTERNATIONALE RESEARCH MAATSCHAPPIJ B.V., 2596 HR Den Haag (NL)
(72) Inventor: MURRAY, Brendan, Dermot, Houston, TX 77077 (US); SINGLETON, David, Michael, Houston, TX 77024 (US)
(86) International application number: EP9504380
(87) International publication number: WO9614280

(56) References cited:
- EP-A- 0 152 314
- EP-A- 0 434 062
- DE-A- 4 206 750
- US-A- 4 305 842
- US-A- 4 386 017
- US-A- 5 334 779
- DATABASE WPI Derwent Publications Ltd., London, GB; AN 94-291776 'Catalyst for hydrogenation of furfural to furfuryl alcohol ' & RO,A,106 848 (COMB. CHIM. VICTORIA ET AL) , 30 July 1993

## Description

This invention relates to a process for the direct hydrogenation of carboxylic esters in a predominantly liquid phase using a catalyst comprising a copper compound, a zinc compound, and at least one compound selected from the group consisting of aluminium, zirconium, magnesium, a rare earth and mixtures thereof.

The hydrogenation of carboxylic acids and carboxylic esters to alcohols is known in the art, and various methods and catalysts have been suggested for effecting the hydrogenation. A commonly practised method involves the use of a copper-chromite-based hydrogenation catalyst. While copper chromite catalysts are successful and commercially available, the disposal of the spent copper chromite catalyst is a problem since chromium can exist in different oxidation states. Some of these oxidation states are reported to be toxic to humans.

From DE-A 42 06 750, US-A-5,334,779 and EP-A 0 434 062 catalysts and the use thereof in the hydrogenation of carboxylic esters are known. These catalysts are based on oxides of copper, zinc, and a third element selected from the group consisting of aluminium, magnesium, zirconium and mixtures thereof.

According to DE-A 42 06 750, the temperature at which the catalyst is calcined is between 250 and 450 °C, and the pressure under which the hydrogenation reaction is to be performed is between 18-35 MPa (equals 181-351 bar gauge).

According to US-A-5,334,779 the catalysts are prepared by calcining a precipitate of the (intermediate) catalyst components at temperatures in the range of 400 to 650 °C. However, hydrogen pressures in the upper part of the pressure range of 68 to 170 bar absolute, e.g., 163 bara, are still required for a reasonable activity. Catalysts having a wider operating window, allowing hydrogenation at lower pressures hence remain desirable.

According to EP-A 0 434 062 the temperature at which the catalyst is calcined is between about 300 and about 550 °C and the pressure under which the hydrogenation reaction is to be performed is broadly between 200-2000 psig (equals 13.8-138 bar gauge). However, this document specifies that the hydrogenation reaction has to be performed in the vapour phase.

The present invention provides a process for the direct hydrogenation of carboxylic esters which comprises contacting and reacting one or more esters with hydrogen in the presence of a hydrogenation catalyst comprising as its components a copper compound, a zinc compound and at least one compound selected from the group consisting of aluminium, zirconium, magnesium, a rare earth and mixtures thereof, the catalyst having been prepared by calcining said components at a temperature in the range of from 200 to less than 400 °C, characterized in that the process is carried out in the liquid phase at a temperature in the range of from 170 to 250 °C and a pressure in the range of from 20.7 to 138 bar gauge.

The present invention relates to a process for the direct hydrogenation of carboxylic esters to form fatty alcohols. Suitable carboxylic ester comprise (i) detergent range methyl esters, (ii) triglycerides and (iii) wax esters.

As used herein, the term "detergent range methyl esters" refers to compounds, typically C₆ to C₂₂ compounds, which have been produced by ester exchange of natural oils, such as coconut oil, rape seed oil, and palm oils. Examples of detergent range methyl esters include methyl decanoate (methyl caprate), methyl dodecanoate (methyl laurate), methyl tetradecanoate (methyl myristate), methyl hexadecanoate (methyl palmitate), methyl octadecanoate (methyl stearate), and methyl octadecenoate (methyl oleate).

As used herein, the term "triglycerides" refers to natural triglycerides, typically C₆ to C₆₆ compounds, which occur as oils and fats in plants and animals. Examples of triglycerides which can be used as starting materials in the process of the present invention include natural oils, including genetically engineered oils, such as, for example, palm kernel oil, coconut oil, rape seed oil, tallow, and palm oil, and vegetable oils, such as, for example, soybean oil, safflower oil, canola oil, corn oil, peanut oil.

As used herein, the term "wax esters" refers to compounds, typically C₆ to C₄₄ compounds, which have been produced by ester exchange of natural triglycerides. Examples of triglycerides which can be used as raw materials to form wax esters include natural oils, such as coconut oil, rape seed oil, and palm oils, and animal fats, such as lard, tallow and fish oil. Examples of wax esters include decyl decanoate (capryl caprate), dodecyl dodecanoate (lauryl laurate), tetradecyl tetradecanoate (myristyl myristate), hexadecyl hexadecanoate (palmityl palmitate), octadecyl octadecanoate (stearate or isostearate), octadecenyl octadecenoate (oleate, linoleate or linolenate), lauryl myristate, myristyl palmitate, oleyl laurate, and docosyl docosanoate (erucyl erucate).

As used herein, "fatty alcohol" refers to an alcohol, preferably a linear alcohol, containing from about 6 to about 22 carbon atoms, preferably from about 10 carbon atoms to about 22 carbon atoms. Typical fatty alcohols produced by the direct hydrogenation process of the present invention include decanol, dodecanol, tetradecanol, hexadecanol, octadecanol, and the like, and mixtures thereof.

The catalyst used in the process of the present invention comprises a mixture of a copper compound, a zinc compound, and at least one compound selected from the group consisting of aluminium, zirconium, magnesium a rare earth and mixtures thereof, prepared by calcining these compounds at a temperature in the range of 200 to less than 400 °C.

It has been found that high purity alcohols can be obtained using a catalyst comprising a copper compound, a zinc compound, and at least one rare earth compound. Such catalyst is therefore preferred.

The metal content of the catalyst is calculated in percent by weight (%wt) as the oxide, based on the total weight of the catalyst. All ratios specified herein are metal atoms unless otherwise noted.

The copper content of the catalyst can vary over a wide range for example, from 10 to 80 %wt. However, for an optimal combination of initial catalyst activity and catalyst stability, a copper content in the range of from 25 to 75 %wt is preferred, especially 30 to 70 %wt.

The zinc content of the catalyst is typically in the range of from 10 to 80 %wt. Preferably, the zinc content of the catalyst is in the range of from 15 to 75 %wt, especially from 20 to 70 %wt. The ratio of zinc to copper in the catalyst is generally in the range of from 1:5 to 5:1, and preferably in the range of from 1:4 to 2:1.

The catalyst additionally comprises at least one compound selected from the group consisting of aluminium, zirconium, magnesium, a rare earth and mixtures thereof.

When a rare earth compound is used, the rare earth content of the catalyst is typically in the range of from 0.1 to 20 %wt. Preferably, the rare earth content of the catalyst is in the range of from 0.2 to 15 %wt, especially from 0.3 to 10 %wt.

As used herein, the terms "rare earth" and "lanthanide" refer to the series of elements with atomic numbers ranging from 57 (lanthanum) through 71 (lutetium). With regard to the rare earth (lanthanide) series, mixed metals are readily available commercially. For purposes of the present invention, the rare earth is selected from the group consisting of praseodymium, neodymium, yttrium, lanthanum, samarium, thorium, cerium and mixtures thereof, with lanthanum being preferred.

When the catalyst contains aluminium and/or zirconium and/or magnesium the aluminium and/or zirconium and/or magnesium total content of the catalyst is typically in the range of from 0.05 to 30 %wt. Preferably, the aluminium and/or zirconium and/or magnesium content of the catalyst is in the range of from 0.4 to 20 %wt, especially from 0.6 to 10 %wt.

In one embodiment, the catalyst comprises copper, zinc and zirconium. In another embodiment, the catalyst comprises, copper, zinc and aluminium. In another embodiment, the catalyst comprises copper, zinc, aluminium and zirconium. In another embodiment, the catalyst comprises, copper, zinc and a rare earth. In another embodiment, the catalyst comprises copper, zinc, magnesium and a rare earth.

Various procedures can be used to prepare the catalysts of the present invention. For example, individual solutions of the metals may be prepared and mixed together followed by the addition of an aqueous alkaline solution. Alternatively, a first aqueous solution comprising a copper or zinc salt and a second solution comprising a soluble base and at least one soluble salt of at least one second metal can be prepared, and these two solutions are then added simultaneously to a vessel containing water. In a preferred embodiment, the catalysts are prepared by co-precipitating from aqueous solution thermally decomposable compounds of copper, zinc, and rare earth and/or aluminium and/or zirconium and/or magnesium, washing the precipitate and calcining the precipitate to give the metal oxides. The catalyst precursor is subjected to a reduction treatment to give the active catalyst.

It is understood that the catalyst is usually handled and stored in the form of its precursor, which indeed is referred to in commerce as the "catalyst", although it is not the catalyst in the strict sense of the agent taking part in chemical reactions such as hydrogenation of carboxylic esters. Reduction of the precursor to the catalyst is normally carried out by the operator of the chemical process. The precursor may be in shapes, e.g., pellets, as required by the user of the catalyst, or may be in its condition before the shaping operation, e.g., as powder or lightly compressed powder.

The initial form in which the copper, zinc and rare earth and/or aluminium and/or zirconium and/or magnesium are employed is the oxide, although compounds which are readily converted to the oxide, e.g., the corresponding metal carbonates, are also suitable initially employed as these are converted to the oxide during pretreatment subsequent to the formation of the initially prepared catalyst composition. Pretreatment of the catalyst in hydrogen and operation of the catalyst in the reaction environment will cause at least partial reduction of some of the metals, such as copper, to lower oxidation states, and it is intended that catalysts with these reduced states will fall within the scope of this invention.

In the method of making the catalyst the reaction conditions for the precipitation should be carefully controlled. The temperature for the precipitation is preferably in the range of from 20 to 100 °C, preferably from 50 to 85 °C, and the pH during the precipitation process is maintained between 5.5 and 7.5, preferably between 6.0 to 7.0 and more preferably, between 6.3 and 6.7.

The precipitating agent may be an alkali metal or an ammonium carbonate solution. The precipitate thus obtained is a mixture of carbonates, basic carbonates, oxides, hydrated oxides and hydroxides. The precipitate is washed, preferably several times with water, aged, reslurried and then dried and calcined, preferably in air at a temperature of from 200 to less than 400 °C, with a temperature of 250 to 350 °C being preferred. It has been established that calcining at a higher temperature yields a catalyst of inferior properties.

The drying is carried out at a temperature sufficient to remove the water. This step is conveniently combined with the calcination by a suitable ramping of the temperature from room temperature slowly through the drying temperature, then up to calcination temperature. The calcined material is shaped, for example, by pelleting under pressure using alumina as a binder, or graphite as lubricant. The oxide mixture is pretreated in a hydrogen-containing atmosphere prior to use as a catalyst to bring it to its most active state. Pretreatment is accomplished by contacting the catalyst with a stream of hydrogen, or of hydrogen mixed with an inert gas or diluent at a temperature ranging from 100 to 400 °C. Suitable diluent gases for the activating gas mixture include nitrogen, the noble gases and mixtures thereof.

In a preferred embodiment, an aqueous solution of copper, zinc and lanthanum salts is employed. Preferably used are copper nitrate, zinc nitrate, and lanthanum nitrate. A second solution of alkali metal or preferably, ammonium carbonate is prepared. The two solutions are heated to a temperature of 20 to 85 °C and simultaneously metered into the precipitation container at rates such that the pH in the precipitation container is maintained between 5.5 and 7.5. Additional water may be used either initially in the precipitation container or added concurrently with the salt solution and precipitation solution. The resulting precipitate is thoroughly washed, dried, calcined at about 300 °C and activated in hydrogen at temperatures ranging from 100 to 400 °C.

In the process of the present invention, one or more carboxylic esters is contacted and reacted with hydrogen in the presence of the above-described catalyst in a reactor. The reactor may be a trickle bed reactor, fixed bed gas-solid reactor, packed bubble column reactor, continuously stirred tank reactor or a slurry phase reactor. The process may be carried out batchwise or in continuous fashion. The reaction is carried out in a predominantly liquid phase. As used herein, the term "predominantly liquid phase" refers to a reaction in which greater than 50%, and approaching 100% of the reaction mixture (other than hydrogen) is in the liquid phase.

The reaction temperatures are typically in the range of from 170 to 250 °C, preferably in the range of from 190 to 240 °C, and more preferably in the range of from 205 to 230 °C. With respect to the reaction temperatures, it is important to ensure that the temperature does not 250 °C. At temperatures greater than 250 °C, the paraffin make increases to unacceptable levels. The reaction pressures are typically in the range of from 20.7 to 138 bar gauge (300 to 2000 psig), preferably in the range of from 27.6 to 103 bar g (400 to 1500 psig), and more preferably in the range of from 34.5 to 68.9 bar g (500 to 1000 psig). Operation at these low reaction pressures is possible due to the high activity and selectivity of the catalysts. The molar ratio of hydrogen to methyl carboxylic ester in the process of the present invention is in the range of from 20:1 to 700:1, preferably from 50:1 to 650:1, and more preferabiy, from 100:1 to 600:1. The process is generally carried out at a weight hourly space velocity (WHSV) in the range of from 0.1 hr⁻¹ to 5 hr⁻¹, preferably in the range of from 0.1 hr⁻¹ to 3 hr⁻¹. The time period required for reaction will vary according to the temperature used, the molar ratio of hydrogen to carboxylic ester, and the partial pressure of hydrogen.

The products produced by the present process include fatty alcohols, (unreacted) wax esters, glycerol, traces of paraffins and unreacted esters. The products are typically separated by conventional means such as, for example, distillation, extraction, filtration, crystallisation, and the like. The desired fatty alcohol products are then used in various applications, such as, for example, detergents such as laundry powders, laundry liquids, etc. and personal care products such as shampoos, etc.

An advantage of the present process is the conversion, i.e., greater than at least about 80 mole %, and approaching 100%, based on the carboxylic ester, as well as the selectivity to alcohols, i.e., greater than 95 mole %, and approaching 100%. As used herein "selectivity" is understood to refer to the selectivity following separation of wax esters, i.e., myristyl myristate, which are recycled. The process also has advantages in that it produces very pure alcohols with very low levels of paraffins at relatively low hydrogen to feed ratios.

Another advantage of the process is the selectivity to linear alcohols, which is typically approaches 100%. The process also has an advantage in that the process allows for the direct conversion of natural oils into linear alcohols while producing very pure alcohols.

The ranges and limitations provided in the present specification and claims are those which are believed to particularly point out and distinctly claim the present invention. It is, however, understood that other ranges and limitations which perform substantially the same function in substantially the same manner to obtain the same or substantially the same result are intended to be within the scope of the present invention as defined by the specification and claims.

The invention is further described with reference to the following examples, which are intended to illustrate certain aspects of the invention, without limiting its broader scope.

### Illustrative Embodiments

### Catalyst Preparation

### Example 1

A solution of mixed nitrates, 96 grams (0.41 mole) of cupric nitrate, 60 grams (0.2 mole) zinc nitrate, 3.3 grams (0.008 mole) lanthanum nitrate in one litre of water was heated to 85 °C and placed in a dish and funnel. In a second funnel was placed a hot 50 °C 1 molar solution of ammonium carbonate of sufficient quantity to provide an excess over the metal nitrates. Two solutions were added simultaneously over a period of about 20 minutes to a vessel containing 1 litre of vigorously stirred distilled water at 65 °C. The rates of addition were adjusted so as to maintain the pH of the mixture at about pH equal to about 6.5. After the addition had been completed the slurry was aged at 85 °C for 20 minutes and then allowed to settle and washed 5 times by decantation and reslurrying before being filtered and dried. The mixed carbonates were then calcined at 300 °C for 4 hours. The resulting oxide material was compressed isostatically at 140 MPa (20,000 lbs) and then crushed and sieved.

### Example 2

The catalyst preparation procedure for Example 1 was followed, except that 3.47g (0.008 mole) cerous nitrate was used in place of the lanthanum nitrate.

### Example 3

The catalyst preparation procedure for Example 1 was followed, except that 3.00g (0.008 mole) zirconyl nitrate was used in place of the lanthanum nitrate.

### Example 4

The catalyst preparation procedure for Example 1 was followed, except that 2.2g (0.009 mole) magnesium nitrate hexahydrate was added to the nitrate salt solution.

### Example 5

A Cu/Zn/Zr catalyst was prepared as in Example 3, the catalyst was then mixed with 10% by weight of Catapal D alumina (marketed by Vista Chemical Corporation) and a small amount of acetic acid as a peptising agent. The mixture was mulled, then extruded and calcined as described in Example 1.

### Example 6

The catalyst preparation procedure for Example 1 was followed, except that 120g (0.55 moles) copper nitrate was used, and the lanthanum nitrate as replaced with 12.0g (0.3 moles) aluminium nitrate nonahydrate.

### Catalyst Testing

The catalytic testing was conducted in a typical laboratory scale reactor. The reactor tube used was constructed of 316 stainless steel and included a thermowell. The tube had an outer diameter of 2.5 cm (1 inch) and an inner diameter of 1.5 cm (0.6 inches). An equal volume of silicon carbide, (60-80 mesh) was mixed with 20.00 g of catalyst and centred in the reactor tube between two beds of enough 20 mesh silicon carbide to fill the reactor. The silicon carbide was used as a diluent. The reactor tube was placed in a four-zone furnace equipped with controlling thermocouples and its fittings were tightened. A multi-point thermocouple was inserted in to the thermowell to allow precise monitoring of the temperatures inside the reactor tube.

The catalyst was reduced by initiating a 10.0 L/Hr flow of approximate 5% hydrogen in nitrogen at a unit pressure of 9.3 bar g (135 psig). The reactor was heated at a rate of 60 °C/Hr to 200 °C. The catalyst was held at 200 °C for 17 hours. While maintaining the same flow rate, the catalyst was next reduced with hydrogen for an additional 8 hours.

After reduction, the unit pressure was raised to the desired pressure, (e.g. 41.4 bar g) by adjusting a back pressure regulator. The hydrogen flow was adjusted to the desired flow rate. The reactor temperature was adjusted to the desired setpoint and the catalyst bed temperatures were allowed to equilibrate before the feed was introduced at the desired feed rate. The feed was stored under nitrogen and, if necessary, heated to a temperature above its melting point before being pumped to the reactor through heated insulated lines to insure proper flow. Typical operating conditions investigated were; temperatures of 150-260 °C; WHSV of 0.1 to 5 per hour; molar hydrogen to feedstock ratios of between 20:1 and 1000:1; and unit pressures between 13.8 138 bar g (200 and 2000 psig). Samples of the feed and products were analysed by GC, NMR, IR, Mass Spectroscopy and elemental analysis.

The results for hydrogenation of pure methyl tetradecanoate (methyl myristate) are shown in Table 1. The results for hydrogenation of coconut oil, employing the catalyst of Example 4, are shown in Table 2. The results for hydrogenation of pure tetradecyl tetradecanoate (myristyl myristate), employing the catalyst of Example 1, are shown in Table 3.

**TABLE 1**

| Temp. (°C) | H₂:Feed | Conversion (%m) | Alcohol (%m) | Paraffin (%m) | Wax (%m) | Purity (%m) |
|---|---|---|---|---|---|---|
| | | | | | | |

| Example 1*: | | | | | | |
|---|---|---|---|---|---|---|
| 204 | 125:1 | 97.3 | 75.8 | 0.4 | 23.8 | 95.9 |
| 221 | 125:1 | 99.7 | 84.8 | 0.6 | 14.4 | 98.8 |
| 227 | 200:1 | 99.9 | 86.9 | 1.0 | 12.5 | 98.6 |

| Example 2*: | | | | | | |
|---|---|---|---|---|---|---|
| 204 | 125:1 | 99.7 | 63.4 | 0.04 | 36.5 | 98.4 |
| 227 | 200:1 | 99.9 | 86.5 | 0.7 | 12.8 | 99.1 |

| Example 3*: | | | | | | |
|---|---|---|---|---|---|---|
| 205 | 125:1 | 99.0 | 73.9 | 0.1 | 26.0 | 98.4 |
| 227 | 200:1 | 99.9 | 87.3 | 0.8 | 11.9 | 98.9 |

| Example 4*: | | | | | | |
|---|---|---|---|---|---|---|
| 205 | 125:1 | 99.4 | 70.7 | 0.1 | 28.9 | 98.5 |
| 228 | 200:1 | 99.9 | 90.1 | 0.9 | 8.9 | 99.8 |

| Example 5*: | | | | | | |
|---|---|---|---|---|---|---|
| 204 | 125:1 | 99.9 | 87.0 | 0.6 | 12.4 | 99.3 |
| 227 | 200:1 | 99.9 | 93.7 | 1.4 | 4.7 | 98.2 |

| Example 6*: | | | | | | |
|---|---|---|---|---|---|---|
| 205 | 125:1 | 99.9 | 87.0 | 0.6 | 12.4 | 99.3 |
| 222 | 125:1 | 99.9 | 93.7 | 1.4 | 4.7 | 98.2 |

| | | | | | | |
|---|---|---|---|---|---|---|
| ***WHSV = 0.4, pressure = 41.4 bar gauge (600 psig)** | | | | | | |

**TABLE 2**

| Product Yield (%wt) | | | | |
|---|---|---|---|---|
| Time (hrs.) | Temp. (°C) | H₂:Feed (molar) | C₈-C₁₈ Linear Alcohols | Heavy Products |
| 1505-1603 | 215.6 | 335:1 | 87.6 | 12.4 |
| 1627-1653 | 226.7 | 335:1 | 92.9 | 7.1 |
| 1673-1777 | 226.7 | 523:1 | 96.6 | 3.4 |
| 1802-1970 | 215.6 | 523:1 | 89.2 | 10.8 |
| 1994-2428 | 221.1 | 523:1 | 94.9 | 5.1 |

**TABLE 3**

| Temperature (°C) | H₂:Feed (molar) | WHSV | Conversion (% m) | Selectivity (% m) |
|---|---|---|---|---|
| 215.6 | 250:1 | 0.15 | 83.0 | 98.7 |
| | 585:1 | 0.15 | 95.2 | 98.7 |
| 226.7 | 250:1 | 0.151 | 86.3 | 97.1 |
| | 585:1 | 0.151 | 99.3 | 97.0 |

As can be seen in Table 1 the present process results in conversions greater than 97 mole % and product purities (of alcohol) greater than 95 mole %. It can also be seen that the product yields of alcohol are greater than 95 mole %, and that the paraffin make is less than 2 mole %. As can be seen in Table 2, the present process results in product yields of linear alcohols which are greater than 85 %wt. As can be seen in Table 3, the present process results in conversions greater than 80 mole% and selectivities greater than 95 mole %. It can also be seen than 95 mole %. It can also be seen that conversion is dependent on H₂:Feed ratio and selectivity is lower at higher temperatures. Both conversion and selectivity approach 100 mole% at suitable conditions.

## Claims

1. A process for the direct hydrogenation of carboxylic esters which comprises contacting and reacting one or more esters with hydrogen in the presence of a hydrogenation catalyst comprising as its components a copper compound, a zinc compound and at least one compound selected from the group consisting of aluminium, zirconium, magnesium, a rare earth and mixtures thereof, the catalyst having been prepared by calcining said components at a temperature in the range of from 200 to less than 400 °C, characterized in that the process is carried out in the liquid phase at a temperature in the range of from 170 to 250 °C and a pressure in the range of from 20.7 to 138 bar gauge.

2. A process as claimed in claim 1, wherein prior to the calcining, the catalyst components are precipitated at a temperature in the range of 20 to 100 °C and a pH between 5.5 and 7.5.

3. A process as claimed in claim 1 or 2, wherein the calcining of the catalyst is at a temperature in the range of 250 to 350 °C.

4. A process as claimed in any one of claims 1 to 3, wherein the catalyst contains copper in the range of from 10 to 80 percent by weight, calculated as the oxide, based on the total weight of the catalyst.

5. A process as claimed in any one of claims 1 to 4, wherein the catalyst contains zinc in the range of from 10 to 80 percent by weight, calculated as the oxide, based on the total weight of the catalyst.

6. A process as claimed in any one of claims 1 to 5, wherein the catalyst comprises at least one rare earth compound.

7. A process as claimed in claim 6, wherein the catalyst contains a rare earth in the range of from 0.1 to 20 percent by weight, calculated as the oxide, based on the total weight of the catalyst.

8. A process as claimed in any one of claims 1 to 7, wherein the carboxylic ester is a C₆ to C₂₂ methyl ester which has been produced by ester exchange of a natural oil, a C₆ to C₆₆ natural triglyceride, or a C₆ to C₄₄ compound which has been produced by ester exchange of a natural triglyceride.

## Patentansprüche

1. Verfahren zur direkten Hydrierung von Carbonsäureestern, welches ein Inkontaktbringen und Umsetzen eines oder mehrerer Ester mit Wasserstoff in Gegenwart eines Hydrierkatalysators umfaßt, der als seine Komponenten eine Kupferverbindung, eine Zinkverbindung und wenigstens eine Verbindung, ausgewählt aus der aus Aluminium, Zirkon, Magnesium, einem Seltenerdmetall und deren Gemischen bestehenden Gruppe, umfaßt, wobei der Katalysator durch Kalzinieren dieser Komponenten bei einer Temperatur im Bereich von 200 bis weniger als 400°C hergestellt worden ist, dadurch gekennzeichnet, daß das Verfahren in flüssiger Phase bei einer Temperatur im Bereich von 170 bis 250°C und bei einem Druck im Bereich von 20,7 bis 138 bar Überdruck ausgeführt wird.

2. Verfahren nach Anspruch 1, worin vor dem Kalzinieren die Katalysatorkomponenten bei einer Temperatur im Bereich von 20 bis 100°C und bei einem pH-Wert von 5,5 bis 7,5 ausgefällt werden.

3. Verfahren nach Anspruch 1 oder 2, worin das Kalzinieren des Katalysators bei ener Temperatur im Bereich von 250 bis 350°C erfolgt.

4. Verfahren nach einem der Ansprüche 1 bis 3, worin der Katalysator Kupfer im Bereich von 10 bis 80 Gew.-%, berechnet als das Oxid und bezogen auf das Gesamtgewicht des Katalysators, enthält.

5. Verfahren nach einem der Ansprüche 1 bis 4, worin der Katalysator Zink im Bereich von 10 bis 80 Gew.-%, berechnet als das Oxid und bezogen auf das Gesamtgewicht des Katalysators, enthält.

6. Verfahren nach einem der Ansprüche 1 bis 5, worin der Katalysator wenigstens eine Seltenerdverbindung umfaßt.

7. Verfahren nach Anspruch 6, worin der Katalysator eine Seltenerdverbindung im Bereich von 0,1 bis 20 Gew.-%, berechnet als das Oxid und bezogen auf das Gesamtgewicht des Katalysators, enthält.

8. Verfahren nach einem der Ansprüche 1 bis 7, worin der Carbonsäureester ein C₆ bis C₂₂ - Methylester ist, der durch Esteraustausch eines natürlichen Öls gebildet worden ist, ein C₆ bis C₆₆ natürliches Triglycerid ist oder eine C₆ bis C₄₄ Verbindung ist, die durch Esteraustausch eines natürlichen Triglycerids erhalten worden ist.

## Revendications

1. Procédé d'hydrogénation directe d'esters carboxyliques, caractérisé en ce que l'on met en contact et fait réagir un ou plusieurs esters avec de l'hydrogéne, en présence d'un catalyseur d'hydrogénation comprenant, à titre de ces composants, un composé du cuivre, un composé du zinc et au moins un composé choisi dans le groupe formé par l'aluminium, le zirconium, le magnésium, une terre rare et leurs mélanges, le catalyseur ayant été préparé par la calcination desdits composants à une température qui varie de 200 à moins de 400°C caractérisé en ce que l'on entreprend le procédé en phase liquide et à une température qui varie de 170 à 250°C et sous une pression qui fluctue de 20,7 à 138 bars manométriques.

2. Procédé suivant la revendication 1, caractérisé en ce que préalablement à la calcination, les composants du catalyseur sont précipités à une température qui varie de 20 à 100°C et à un pH compris entre 5,5 et 7,5.

3. Procédé suivant la revendication 1 ou 2, caractérisé en ce que la calcination du catalyseur s'effectue à une température qui varie de 250 à 350°C.

4. Procédé suivant l'une quelconque des revendications 1 à 3, caractérisé en ce que le catalyseur contient du cuivre dans la proportion de 10 à 80% en poids, calculés sous forme de l'oxyde, sur base du poids total du catalyseur.

5. Procédé suivant l'une quelconque des revendications 1 à 4, caractérisé en ce que le catalyseur contient du zinc en une proportion de 10 à 80% en poids, calculés sous forme de l'oxyde, sur base du poids total du catalyseur.

6. Procédé suivant l'une quelconque des revendications 1 à 5, caractérisé en ce que le catalyseur comprend au moins un composé des terres rares.

7. Procédé suivant la revendication 6, caractérisé en ce que le catalyseur contient une terre rare en une proportion de 0,1 à 20% en poids, calculés sous forme de l'oxyde, sur base du poids total du catalyseur.

8. Procédé suivant l'une quelconque des revendications 1 à 7, caractérisé en ce que l'ester carboxylique est un ester méthylique en c₆ à c₂₂, qui a été produit par échange d'esters d'une huile naturelle, d'un triglycéride naturel en C₆ à C₆₆, ou d'un composé en C₆ à C₄₄ qui a été produit par échange d'esters d'un triglycéride naturel.
